# FASCICULE DE BREVET EUROPEEN

(11) **EP 2 496 308 B1**
(45) Date de publication et mention de la délivrance du brevet: **30.05.2018**
(21) Numéro de dépôt: 10799059.0
(22) Date de dépôt: 02.11.2010
(51) Int. Cl.: A61P 17/14, A61K 8/97, A61K 36/899, A61Q 5/00, A61Q 7/00

(54) **UTILISATION D'UN HYDROLYSAT PEPTIDIQUE DE MAÏS EN TANT QU'AGENT ACTIF STIMULANT LA POUSSE DU CHEVEU**
VERWENDUNG EINES PEPTIDHYDROLYSATES VON MAIS ALS AKTIVE SUBSTANZ ZUR STIMULIERUNG VON HAARWUCHS
USE OF A PEPTIDE HYDROLYSATE FROM MAIZE AS ACTIVE AGENT STIMULATING HAIR GROWTH

(30) Priorité: 03.11.2009 FR 0905258
(43) Date de publication de la demande: 12.09.2012
(73) Titulaire: ISP Investments Inc., Wilmington, DE 19805 (US)
(72) Inventeur: DAL FARRA, Claude, Kerhonkson NY 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2010/000723
(87) Numéro de publication internationale: WO 2011/055033

(56) Documents cités:
- WO-A1-2010/119192
- CH-A5- 682 217
- DE-A1- 3 130 894
- FR-A1- 2 904 543
- FR-A1- 2 911 070
- FR-A1- 2 915 382
- GB-A- 2 060 378
- Mouser Paul et al.: "Modulation of hair growth markers in the hair", , 30 avril 2010 (2010-04-30), pages 1-1, XP002580640, Extrait de l'Internet: URL:http://www.hair2010.org/abstract/150.a sp [extrait le 2010-04-30]
- DATABASE BIOSIS [Online] BIOSCIENCES INFORMATION SERVICE, PHILADELPHIA, PA, US; juin 1999 (1999-06), ETUKUDO MAISIE ET AL: "Biochemical changes and liver tissue pathology in weanling Wistar albino rats with protein energy malnutrition (PEM)", XP002580641, Database accession no. PREV200000109217 & DISCOVERY AND INNOVATION, vol. 11, no. 1-2, juin 1999 (1999-06), pages 83-89, ISSN: 1015-079X
- SQUIBB R L ET AL: "A comparison of the effect of raw corn and tortillas (lime-treated corn) with niacin, tryptophan or beans on the growth and muscle niacin of rats.", THE JOURNAL OF NUTRITION 10 MAR 1959 LNKD- PUBMED:13642128, vol. 67, no. 3, 10 mars 1959 (1959-03-10), pages 351-361, XP002580642, ISSN: 0022-3166
- DATABASE WPI Week 198926 Thomson Scientific, London, GB; AN 1989-189472 XP002583774, & JP 1 128912 A (KOKURA) 22 mai 1989 (1989-05-22)
- DATABASE WPI Week 199017 Thomson Scientific, London, GB; AN 1990-129041 XP002583775, & JP 2 078608 A (KUDO T) 19 mars 1990 (1990-03-19)

## Description

### Domaine de l'invention

La présente invention se situe dans le domaine cosmétique et dermato-pharmaceutique. L'invention concerne l'utilisation cosmétique d'une composition comprenant au moins un hydrolysat peptidique de maïs (*Zea mays L.),* en tant qu'agent actif, pour stimuler et/ou restaurer la pousse du cheveu, ou prévenir la chute du cheveu. L'agent actif provient de l'hydrolyse de protéines de plantes choisies parmi les plantes du genre Zea, et plus particulièrement de l'espèce *Zea maïs L.* L'invention est également relative à l'utilisation de ce nouvel agent actif pour la réalisation d'une composition dermato-pharmaceutique, destinée à stimuler et/ou restaurer la pousse du cheveu, ou prévenir la chute du cheveu, dans le cadre d'un traitement préventif ou curatif de la perte de cheveux liée à un état pathologique.

### Arrière plan de l'invention

La croissance des cheveux et leur renouvellement sont principalement déterminés par l'activité des follicules de cheveu et de leur environnement matriciel.

Le follicule de cheveu est une annexe cutanée autonome complexe qui comporte six grands compartiments, les uns d'origine dermique (gaine conjonctive et papille dermique), les autres de nature épithéliale (gaines épithéliales interne et externe, tige pilaire et sébacée). A la base du follicule se trouve la matrice, siège d'une activité mitotique intense, à l'origine des 3 couches concentriques du cheveu constitué essentiellement de kératine.

Le follicule de cheveu se renouvelle *in situ* de façon cyclique et asynchrone à partir d'un double réservoir de cellules souches, selon un cycle d'activité qui comprend trois phases : la phase anagène, la phase catagène et la phase télogène.

La phase anagène (phase de croissance) dure de un à dix ans et se caractérise par l'allongement constant du cheveu. Durant cette phase, les cellules de la gaine épithéliale externe sur-expriment l'intégrine bêta 1 (Commo & Bernard, 1997, Cell mol. life Sc. 53 :466-471). Les cellules prolifératives de cette zone expriment des marqueurs de prolifération tels que la protéine Ki67 et le facteur de transcription p63 (Chikh et al., 2007, Biochem Biophys Res Commun.,14;361(1):1-6).

La phase catagène qui succède est très transitoire et dure quelques semaines. Elle semble initiée par certains facteurs tels l'EGF, le TGF bêta (Foitzik et al. 2000, FASEB J. 14(5):752-60), ou encore la protéine p53 qui est transitoirement surexprimée (Botchkarev et al., 2001, Am. J. Pathol. 158(6): 1913-1919). Au cours de cette phase, les cellules du follicule subissent un processus actif d'apoptose, le follicule s'atrophie et se rétracte vers la surface, à l'exception notoire de la papille dermique qui sera l'élément-clé de la future régénération.

La phase terminale ou phase télogène, qui dure quelques mois, correspond à une phase de repos du follicule à la fin de laquelle le cheveu finit par tomber. A la fin de cette période de repos, un nouveau follicule est régénéré et un nouveau cycle démarre.

Il est par ailleurs bien décrit que les mécanismes de différenciation des kératinocytes de l'épiderme et du follicule de cheveu sont sensiblement différents. Ainsi, il est connu que les kératines de la tige pilaire représentent une famille de kératines distincte de celle exprimée dans l'épiderme (Langbein et al., 2001, J. Biol. Chem. 276), ainsi la kératine K6irs (Porter et al., 2001, Br. J. Dermatol. 145: 558-568) est exprimée dans la gaine interne du follicule pileux mais pas dans l'épiderme, alors que les marqueurs de différenciation épidermiques tels les kératines K1 et K10 ne sont pas exprimées dans le follicule pileux (Lenoir et al., 1988, Dev. Biol. 130: 610-620).

La chute ou perte naturelle des cheveux peut être estimée, en moyenne, à une centaine de cheveux par jour pour un état physiologique normal. Ce processus de renouvellement permanent peut être perturbé par de nombreux facteurs extrinsèques et intrinsèques, entraînant une perte importante, temporaire ou définitive, des cheveux que l'on regroupe sous le terme générique d'alopécie.

Comme le reste de l'organisme, le processus de renouvellement physiologique permanent des cheveux est soumis au vieillissement. Si le signe le plus visible du vieillissement du cheveu est le blanchiment, la qualité de l'environnement biologique du follicule du cheveu est également affectée. Parmi les manifestations de vieillissement du cheveu, on observe une moindre synthèse des protéines de la matrice extracellulaire (collagène, laminine, fibronectine), provoquant une perte d'élasticité et de tonicité du tissu sous-cutané. Il y a également une diminution de la cohérence et de l'organisation des follicules de cheveu, une diminution de la durée de la phase anagène et un allongement de la durée de la phase télogène (Courtois et al., 1995, Br. J. dermatol., 132 :86-93). Les cheveux perdent en effet de leur élasticité, ils sont plus fins donc plus fragiles. Au niveau de l'ensemble du cuir chevelu, le vieillissement se manifeste par une diminution de la densité capillaire et par la diminution progressive du diamètre des follicules, donnant à la chevelure un aspect plus pauvre, plus clairsemé (Pelfini, C. et al., J. Méd. Esth. Et Chir. Demi.1987; Birch MP et al. Br. J. Dermatol 2001;144:297-304).

Indépendamment du vieillissement intrinsèque, des altérations du cheveu ou du follicule de cheveu peuvent se produire lors d'agressions extérieures. En effet, si le cheveu est d'une remarquable stabilité, certains facteurs extérieurs, tels que le soleil, responsable du photo-vieillissement, les radicaux libres, la pollution ou encore certains traitements inappropriés peuvent aboutir à une détérioration prématurée de la structure des cheveux et à un épuisement des follicules.

On entend par l'expression « agression extérieure », les agressions que peut produire l'environnement. A titre d'exemple, on peut citer des agressions telles que la pollution, les UV, ou encore les produits à caractère irritant tels que les tensioactifs trop détergents, les colorations et décolorations, permanentes ou défrisages trop fréquents, les agressions mécaniques telles que le frottement des vêtements, les coiffures provoquant des étirements répétés, le brossage trop intense, les brushings, le séchage à l'air trop chaud. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldéhyde), ou bien encore la fumée de cigarette. Ces agressions extérieures aboutissent à une altération de la structure externe du cheveu et de ses propriétés mécaniques, mais peuvent aussi affecter le follicule de cheveu et provoquer un vieillissement prématuré.

L'industrie cosmétique ou pharmaceutique recherche toujours des compositions permettant de supprimer ou de réduire la perte ou de stimuler la croissance des cheveux. Comme molécule connue, on peut citer le 2,4-diamino 6-piperidinopyrimidine 3-oxyde ou "minoxidil" (brevets US 4 139 619 et US 4 596 812). Un autre document brevet de Shiseido (JP 07316023) décrit également l'utilisation de l'arginine et de ses dérivés dans le traitement de l'alopécie. Enfin, le document EP 1885323 décrit l'utilisation d'un peptide de synthèse pour stimuler la pousse des cheveux.

Toutefois, certains produits disponibles présentent des effets secondaires, comme c'est le cas pour le minoxidil, ou ont une efficacité relative et circonscrite à la durée du traitement. Aussi, il subsiste le besoin d'une nouvelle composition physiologiquement acceptable pour favoriser la croissance des cheveux et/ou réduire leur chute, présentant une action rapide et durable dans le temps.

Des extraits peptidiques de maïs ont précédemment été décrits pour leurs effets sur la peau (FR 2904543, FR2915382, FR2925326).

Les inventeurs ont maintenant mis en évidence que de tels hydrolysats peptidiques de maïs avaient une activité sur le follicule de cheveu. Il a en particulier été démontré que l'hydrolysat peptidique, lorsqu'il est appliqué sur le cheveu, active le follicule de cheveu et stimule la pousse du cheveu.

### Exposé de l'invention

Les objets de l'invention sont définis dans les revendications. Tous les autres objets sont décrits dans un but illustratif, et les indications que ces objets constitueraient l'invention, ou toute indication similaire, doivent être perçues dans le contexte de la demande telle que déposée, ne changeant pas l'étendue de protection des revendications. Le premier objet de la présente invention est l'utilisation cosmétique d'une composition comprenant au moins un hydrolysat peptidique de maïs (*Zea mays L.)* en tant qu'agent actif, pour stimuler et/ou restaurer la pousse du cheveu, ou prévenir la chute du cheveu.

Les fibres kératiniques humaines auxquelles s'applique l'invention sont notamment les cheveux, les sourcils, les cils, les poils de barbe, de moustache, les poils pubiens et les ongles. Plus spécialement, l'invention s'applique aux cheveux et/ou aux cils humains.

On entend par « hydrolysat peptidique », un mélange de composés majoritairement représentés par des peptides ou des oligopeptides. Selon l'invention, on utilisera indifféremment les termes « hydrolysat peptidique » ou « agent actif ».

On entend par « agent actif pour stimuler et/ou restaurer la pousse du cheveu, ou prévenir la chute du cheveu », tout hydrolysat peptidique de maïs (*Zea mays L.),* capable de stimuler le follicule de cheveu, c'est-à-dire d'augmenter l'expression des principales molécules de la matrice extracellulaire telle que la fibronectine, de la lame basale telle que la laminine-5 et des principaux marqueurs intracellulaires de la phase anagène du follicule de cheveu, tels que l'intégrine bêta 1, les marqueurs de prolifération Ki67, p63 et l'histone H3 phosphorylée, ou à contrario, d'inhiber l'expression d'initiateurs de la phase catagène, tel que la protéine p53, permettant ainsi une prolongation de la phase anagène de croissance du cheveu.

L'agent actif selon l'invention peut être obtenu par extraction de protéines d'origine végétale, suivie d'une hydrolyse contrôlée qui libère des composés de nature peptidique.

On entend par « composés de nature peptidique », les fragments de protéines et les peptides présents dans l'hydrolysat peptidique selon l'invention.

L'utilisation d'hydrolysats peptidiques, et en particulier d'hydrolysats peptidiques de bas poids moléculaires, présente de nombreux avantages en cosmétique. Outre le fait de générer des composés de nature peptidique qui ne préexistaient pas dans le mélange protéique de départ, l'hydrolyse et la purification permettent d'obtenir des mélanges plus stables, plus facilement standardisables et ne provoquant pas de réactions allergiques en dermatocosmétique.

De très nombreuses protéines de plantes sont susceptibles de contenir des peptides bioactifs au sein de leur structure. L'hydrolyse ménagée permet de dégager ces composés de nature peptidique. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les composés de nature peptidique ensuite.

Selon un mode de réalisation préférée, ledit agent actif provient de l'hydrolyse de protéines de plantes choisies parmi des plantes du genre Zea et préférentiellement l'espèce *Zea mays L.* Selon l'invention, le matériel végétal utilisé sera le grain et préférentiellement le grain débarrassé de son enveloppe par une étape de décorticage. De préférence, les plantes utilisées ne sont pas soumises à une fermentation préalable.

Toute méthode d'extraction ou de purification connue de l'homme du métier peut être utilisée afin de préparer l'hydrolysat selon l'invention.

Dans une première étape, les graines sont broyées à l'aide d'un broyeur à plantes. La poudre ainsi obtenue peut ultérieurement être "délipidée" à l'aide d'un solvant organique classique (comme par exemple un alcool, de l'hexane ou de l'acétone).

On réalise ensuite l'extraction des protéines suivant le procédé classique (Osborne, 1924) modifié ; le broyat de plante est mis en suspension dans une solution alcaline contenant un produit adsorbant de type polyvinylpolypyrrolidone (PVPP) insoluble (0,01 -20 %) ; en effet, il a été observé que les opérations d'hydrolyses et de purifications ultérieures étaient facilitées par ce moyen. En particulier, la concentration en substances de type phénoliques, interagissant avec les protéines, se trouve nettement réduite.

La fraction soluble, contenant les protéines, les glucides et éventuellement des lipides, est recueillie après des étapes de centrifugation et de filtration. Cette solution brute est ensuite hydrolysée dans des conditions ménagées pour générer des peptides. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques. On peut alors citer l'utilisation des endoprotéases d'origine végétale (papaïne, bromelaïne, ficine) ou de micro-organismes (Aspergillus, Rhizopus, Bacillus, alcalase® de Novozym, etc.).

Pour les mêmes raisons que précédemment, c'est-à-dire l'élimination des substances polyphénoliques, une quantité de polyvinylpolypyrrolidone est additionnée au milieu réactionnel lors de cette étape d'hydrolyse ménagée. Après l'étape de filtration, qui permet d'éliminer les enzymes et les polymères, le filtrat (solution) obtenu constitue une première forme de l'agent actif selon l'invention.

L'hydrolysat obtenu à ce stade peut être encore purifié afin de sélectionner les composés de nature peptidique de poids moléculaire inférieur à 10 kDa. Le fractionnement peut s'effectuer avantageusement par des étapes d'ultrafiltrations successives à travers des filtres de porosité décroissante, en conservant les filtrats à chaque étape. Après cette étape, la majorité des composés de nature peptidique contenus dans l'hydrolysat, et préférentiellement 90 % d'entre eux, ont un poids moléculaire inférieur à 5 kDa.

L'hydrolysat peptidique obtenu selon l'invention est analysé qualitativement et quantitativement pour ses caractéristiques physico-chimiques et sa teneur en composés de nature peptidique. Les peptides et les fragments de protéines sont dosés selon les techniques classiques, bien connues de l'homme du métier.

On procède à une phase de dilution dans de l'eau ou dans tout mélange de solvants contenant de l'eau, afin d'obtenir un hydrolysat peptidique caractérisé par un poids sec de 2 à 5 g/Kg, une concentration en composés de nature peptidique de 1 à 5 g/l, et préférentiellement de 1,5 à 3,0 g/l et une concentration en sucres de 0,05 à 1 g/l et préférentiellement de 0,1 à 0,3 g/l. Les solvants utilisés sont physiologiquement acceptables et classiquement utilisés par l'homme du métier, choisis parmi le glycérol, l'éthanol, le propanediol, le butylène glycol, le dipropylène glycol, les diglycols éthoxylés ou propoxylés, les polyols cycliques ou tout mélange de ces solvants.

Ainsi, l'agent actif selon l'invention est avantageusement solubilisé dans un ou plusieurs solvants physiologiquement acceptables, tels que l'eau, le glycérol, l'éthanol, le propanediol, le butylène glycol, le dipropylène glycol, les diglycols éthoxylés ou propoxylés, les polyols cycliques ou tout mélange de ces solvants. L'agent actif dilué est ensuite stérilisé par ultrafiltration.

Après cette étape de dilution, l'agent actif peut être encapsulé ou inclus dans un vecteur cosmétique ou pharmaceutique tels que les liposomes ou toute autre microcapsule utilisée dans le domaine de la cosmétique ou adsorbé sur des polymères organiques poudreux, des supports minéraux comme les talcs et bentonites.

Selon un mode de réalisation avantageux, l'agent actif est présent dans les compositions de l'invention à une concentration comprise entre 0,001 % et 5 % environ, et préférentiellement à une concentration comprise entre 0,01 % et 1 % environ par rapport au poids total de la composition finale.

Les compositions utilisables selon l'invention peuvent se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la zone à traiter sous forme d'aérosol. Elles peuvent être utilisées comme produit de soin et/ou comme produit de maquillage de la peau.

L'ensemble de ces compositions comprennent, en outre, tout additif communément utilisé dans le domaine d'application envisagé ainsi que les adjuvants nécessaires à leur formulation, tels que des co-solvants (éthanol, glycérol, alcool benzylique, humectants...), des épaississants, des diluants, des émulsionnants, des antioxydants, des colorants, des filtres solaires, des pigments, des charges, des conservateurs, des parfums, des absorbeurs d'odeur, des huiles essentielles, des oligo-éléments, des acides gras essentiels, des tensioactifs, des polymères filmogènes, des filtres chimiques ou minéraux, des agents hydratants ou des eaux thermales etc. On peut par exemple citer des polymères hydrosolubles de type polymère naturel, tels que les polysaccharides, ou polypeptides, des dérivés cellulosiques de type methylcellulose ou hydroxypropylcellulose, ou encore des polymères synthétiques, polaxamères, carbomères, PVA ou PVP et notamment les polymères vendus par la société ISP.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à une concentration allant de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Les compositions utilisables selon l'invention peuvent en particulier consister en un shampooing, un après-shampooing, une lotion traitante pré- ou post- traitement agressif pour les cheveux, une crème ou un gel coiffant, une lotion restructurante pour les cheveux, un masque, etc. La composition peut également se présenter sous forme de mascara pour une application sur les cils, les sourcils ou les cheveux.

Par ailleurs l'agent actif selon l'invention peut être utilisé seul ou bien en association avec d'autres agents actifs.

Avantageusement, les compositions utilisables selon l'invention contiennent, en outre, au moins un autre agent actif protecteur ou qui améliore la pousse et/ou la santé des cheveux. On peut citer, de manière non limitative, les ingrédients suivants : des vitamines, des agents anti-radicalaires, anti-UV, d'autres extraits peptidiques végétaux, le Minoxidil, des esters de l'acide nicotinique, des agents anti-inflammatoires, de l'acide rétinoïque ou ses dérivés, du rétinol, des inhibiteurs de la 5 alpha-réductase ou des composés peptidiques issus de la synthèse chimique.

La composition utilisable selon l'invention pourra être appliquée par toute voie appropriée, notamment orale, parentérale ou topique, et la formulation des compositions sera adaptée par l'homme du métier, en particulier pour des compositions cosmétiques ou dermatologiques.

Avantageusement, les compositions selon l'invention sont destinées à une administration par voie topique. Ces compositions doivent donc contenir un milieu physiologiquement acceptable, c'est-à-dire compatible avec la peau et les phanères, et couvrent toutes les formes cosmétiques ou dermatologiques.

On entend par « application topique », le fait d'appliquer ou d'étaler l'agent actif selon l'invention, ou une composition le contenant, à la surface de la peau ou d'une muqueuse. On entend par « physiologiquement acceptable », que l'hydrolysat peptidique selon l'invention, ou une composition le contenant, est approprié pour entrer en contact avec la peau ou une muqueuse sans provoquer de réactions de toxicité ou d'intolérance.

Selon un autre aspect de l'invention la composition comprenant l'hydrolysat peptidique de maïs (*Zea mays L.),* en tant qu'agent actif, est utilisée pour lutter contre l'alopécie.

L'alopécie recouvre un ensemble d'atteintes du follicule pileux ayant pour conséquence finale la perte transitoire ou définitive, partielle ou générale des cheveux. Les hommes comme les femmes peuvent être atteints d'alopécie, mais les zones préférentiellement touchées chez les hommes sont les golfes temporaux ou frontaux, alors que chez les femmes, on constate une alopécie diffuse du vertex.

Le deuxième objet de l'invention est l'utilisation d'un hydrolysat peptidique de maïs (*Zea mays L.),* en tant qu'agent actif capable de stimuler le follicule de cheveu.

Les inventeurs ont en effet mis en évidence que l'hydrolysat peptidique de maïs (*Zea mays L.)* permettait de stimuler le follicule de cheveu, ce qui se traduisait au niveau moléculaire par l'augmentation de l'expression des molécules de la matrice extracellulaire, telle que la fibronectine, des protéines de la lame basale, telle que la laminine-5. L'action de l'hydrolysat peptidique de maïs (*Zea mays L.)* se traduit également par l'augmentation de l'expression des protéines membranaires impliquées dans les interactions entre les kératinocytes (interactions cellules-cellules) et entre les kératinocytes et la matrice extracellulaire (interactions cellule-matrice), telle que l'intégrine bêta 1. La stimulation du follicule de cheveux se traduit également par l'augmentation de l'expression de marqueurs intracellulaires de la phase anagène, tels que les marqueurs de prolifération Ki67, p63 et l'histone H3 phosphorylée, ou à contrario, par l'inhibition de l'expression d'initiateurs de la phase catagène, tel que la protéine p53, permettant ainsi une prolongation de la phase anagène de croissance du cheveu.

Au niveau organique, la stimulation du follicule de cheveu se manifeste par l'élongation du cheveu dans un système de culture *ex vivo.*

Le troisième objet de l'invention est l'utilisation d'un hydrolysat peptidique de maïs (*Zea mays L.),* en tant qu'agent actif capable de stimuler le follicule de cheveu, pour la réalisation d'une composition dermato-pharmaceutique destinée à stimuler et/ou à restaurer la pousse du cheveu ou à limiter la chute du cheveu, dans le cadre d'un traitement préventif ou curatif de la perte de cheveux liée à un état pathologique. Parmi les états pathologiques fréquemment responsables de la perte de cheveux, on peut citer la pelade, les effets secondaires de traitements médicamenteux, certaines infections ou inflammations du cuir chevelu (le psoriasis, la dermatite séborrhéique, etc...).

Selon cette forme de l'invention, les compositions seront appropriées à une administration orale pour un usage pharmaceutique. Ainsi, les compositions pourront notamment se présenter sous forme de comprimés, capsules, gélules, pâtes à mâcher, poudres à consommer telles quelles ou à mélanger extemporanément avec un liquide, sirop, gels, et toute autre forme connue de l'homme du métier. Ces compositions comprennent, en outre, tout additif communément utilisé dans le domaine d'application envisagé ainsi que les adjuvants nécessaires à leur formulation, tels que des solvants, des épaississants, des diluants, des antioxydants, des conservateurs, d'autres agents actifs pharmaceutiques, des huiles essentielles, des vitamines, des acides gras essentiels, etc.

Des modes de réalisation particuliers de ce procédé de traitement cosmétique résultent également de la description précédente. D'autres avantages et caractéristiques de l'invention apparaîtront mieux à la lecture des exemples donnés à titre illustratif et non limitatif.

### Liste des figures

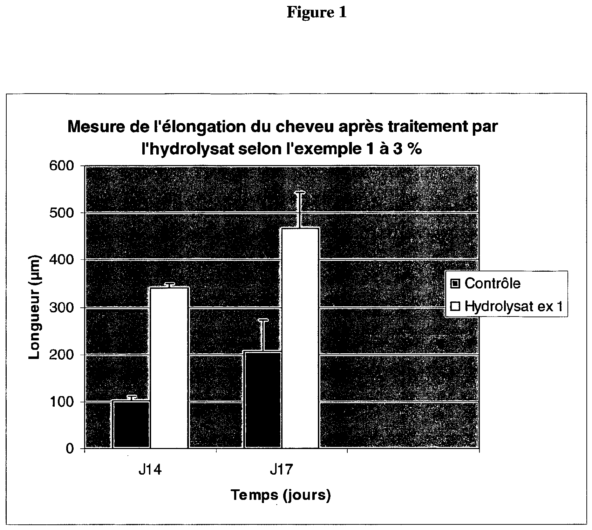

Figure 1 : Mesure quantitative de l'élongation du cheveu après traitement par l'hydrolysat selon l'exemple 1 à 3 %.

### Exemple 1 : Préparation d'un hydrolysat peptidique à partir de tourteaux de maïs (Zea mays L.)

Le tourteau de maïs (*Zea mays L*.) est mis en solution dans 10 volumes d'eau en présence de 2 % de POLYCLAR® 10 (polyvinylpyrrolidone - PVPP - insoluble). Le mélange est ajusté à un pH compris entre 7,5 et 9,5 avec une solution aqueuse de soude 1 M.

Après ajustement du pH, un mélange de bromélaïne à 2 % et d'alcalase® (Novozym) est ajouté dans le milieu réactionnel. L'hydrolyse est obtenue après 2 heures d'agitation à 50°C. On procède ensuite à l'inactivation des enzymes par chauffage de la solution à 80°C pendant 2 heures. Après centrifugation, la solution aqueuse surnageante correspondant à un hydrolysat brut de maïs est récupérée.

Le procédé de purification de l'hydrolysat brut commence par des filtrations successives à l'aide de filtres-plaques Seitz-Orion de porosité décroissante (jusqu'à 0,2 µm) afin d'obtenir une solution jaune, brillante et limpide, qualifié d'hydrolysat 1.

A cette étape, l'hydrolysat 1 de maïs est caractérisé par un extrait sec titrant de 20 à 30 g/kg, un taux de protéines de 20 à 25 g/l et un taux de sucres de 2 à 5 g/l.

La nature protéique de l'hydrolysat 1 est mise en évidence après analyse par électrophorèse sur gel de polyacrylamide NuPAGE® Bis-Tris Pre-cast (Invitrogen). L'hydrolysat protéique de maïs est chauffé à 70°C pendant 10 minutes, dans des conditions réductrices dénaturantes (tampon de préparation d'échantillon NuPAGE® LDS). Une solution de NuPAGE® Antioxydant est ajoutée dans la cuve intérieure (cathode) pour éviter que les protéines réduites ne se ré-oxydent durant l'électrophorèse. La migration des protéines est réalisée dans du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe que 100 % des composés de nature peptidique ont un poids moléculaire inférieur à 10 kDa.

L'hydrolysat 1 est ensuite purifié en éliminant les protéines de haut poids moléculaire par ultrafiltration à l'aide de la cassette Pellicon® 2 Biomax 5 kDa. Après cette étape, 90 % des composés de nature peptidique de l'hydrolysat ont un poids moléculaire inférieur à 5 kDa.

Après cette purification finale, on obtient un hydrolysat ayant un poids sec de 9,7 g/Kg ; 6,2 g/l de composés de nature peptidique et 0,6 g/l de sucres. On procède alors à une phase de dilution dans un mélange eau-glycérol afin d'obtenir un hydrolysat peptidique caractérisé par un poids sec de 2 à 5 g/Kg, une concentration en composés de nature peptidique de 1,5 à 3,0 g/l et une concentration en sucres de 0,1 à 0,3 g/l. Cet hydrolysat peptidique correspond à l'agent actif selon l'invention.

Cet hydrolysat peptidique est alors analysé en chromatographie liquide haute pression (HPLC) à l'aide d'un appareil HP1100 piloté par le logiciel ChemStation. La colonne utilisée lors de l'élution de l'hydrolysat 2 est une Nucleosil® 300-5 C4 MPN (125 x 4 mn) permettant de chromatographier des protéines ayant des poids moléculaires de 0.2 à 25 kDa (selon un gradient de solvants approprié). Dans ces conditions chromatographiques, plusieurs fractions peptidiques ont pu être identifiées.

La détermination de la composition en acides aminés de l'agent actif selon l'invention a également été réalisée. Après un dosage des protéines et des peptides par la méthode de Lowry, une hydrolyse acide est réalisée, pour réduire tous les peptides à l'état d'acides aminés libres. Un exemple de composition en acides aminés de l'hydrolysat est donné dans le tableau suivant. Les valeurs sont exprimées en pourcentage d'acides aminés pour 100 g de protéines.

| Acides aminés | % |
|---|---|
| Alanine | 9,4 |
| Acide Aspartique | 7,2 |
| Arginine | 3,6 |
| Acide Glutamique | 23,7 |
| Glycine | 3,6 |
| Histidine | 2,2 |
| Isoleucine | 4,5. |
| Leucine | 16,1 |
| Lysine | 2,2 |
| Phénylalanine | 6,7 |
| Proline | 10,3 |
| Serine | 6,3 |
| Thréonine | 4,0 |
| Tyrosine | 5,8 |
| valine | 5,4 |
| Tryptophane | < 0,5 |

### Exemple 2 : Mise en évidence de la stimulation de la pousse du cheveu par l'hydrolysat selon l'exemple 1

Le but de cette étude est de déterminer l'influence de l'hydrolysat selon l'exemple 1 sur la pousse du cheveu humain. Pour cela, la mesure de l'élongation de la tige pilaire prolongeant les follicules de cheveu cultivés *ex vivo* a été réalisée.

Protocole : Des punch biopsies de peaux de 6 mm de diamètre provenant de lifting et contenant des follicules de cheveu sont mis en culture sur des inserts dans un milieu WILLIAM E. Au jour 0, les biopsies sont rasées, puis chaque 24 heures les explants de peaux sont traités avec 20 µl d'une solution à 3 % d'hydrolysat selon l'exemple 1, déposés sur la biopsie pendant 14 et 17 jours. Des contrôles non traités sont effectués en parallèle.

Des photos sont réalisées à l'aide de la caméra CCD Vivacam du vivascope 1500® (Europe Mavig) et la longueur des cheveux de chaque follicule est évaluée à l'aide du logiciel « Image Pro Analyser 6.3 ».

Résultats : La longueur des cheveux est exprimée en µm (voir figure 1). En présence de 3% d'hydrolysat selon l'exemple 1, l'élongation du cheveu est multipliée par 3,37 après 14 jours et par 2,26 fois après 17 jours.

Conclusion : L'hydrolysat selon l'exemple 1 stimule très significativement la pousse du cheveu.

### Exemple 3 : Mise en évidence de la stimulation de l'activité cellulaire du follicule de cheveu par l'hydrolysat selon l'exemple 1

Le but de cette étude est de montrer l'effet stimulant sur les fonctions cellulaires,), et en particulier, sur le renouvellement des cellules du follicule de cheveu de l'hydrolysat selon l'exemple 1. Pour ce faire des marqueurs de la prolifération cellulaire (Ki67), du renouvellement épidermique (p63) et de la mitose (histone 3 phosphorylée), ainsi qu'un marqueur de l'entrée en phase catagène, ont été étudiés.

Protocole : Des biopsies de peau humaine sont mises en culture dans les mêmes conditions que dans l'exemple 2, puis traitées pendant 48 heures par l'hydrolysat selon l'exemple 1 à 1 %. Des contrôles non traités sont également réalisés. A la fin de l'expérience, les biopsies sont incluses dans de la résine OTC et congelées dans l'azote. Des coupes d'environ 6 µm sont ensuite réalisées au cryostat. Les coupes sont recueillies sur des lames d'observation poly-lysinées, puis les coupes sont fixées dans un bain d'acétone (conservé au préalable à -20°C) pendant 10 minutes. L'immunomarquage est réalisé à l'aide d'un anticorps polyclonal anti-Ki67 (Abcam) ou d'un anticorps polyclonal anti-Histone 3 phosphorylée (Abcam) ou d'un anticorps monoclonal anti-p63 (Tebu, SantaCruz) ou d'un anticorps monoclonal anti-p53 (Dako). Un anticorps secondaire adapté, couplé à un marqueur fluorescent est ensuite mis en contact avec les coupes. Les coupes de follicules sont enfin examinées au microscope à Epi-fluorescence (Nikon Eclipse E 80i microscope).

Résultats : Sur les coupes de follicule de cheveu traitées par l'hydrolysat selon l'exemple 1, on observe qu'un nombre plus important de cellules de la gaine épithéliale externe sont marquées pour Ki67, l'histone 3 phosphorylée et p63. Au contraire les cellules marquées par l'anticorps p53 sont moins nombreuses en comparaison avec le contrôle non traité.

A l'aide d'un logiciel de quantification de la fluorescence, on peut mesurer une augmentation de 18,5 % pour Ki67, de 7,1 % pour l'histone 3 phosphorylée et de 19,1% pour p63.

Conclusions : L'hydrolysat selon l'exemple 1 augmente l'expression des marqueurs de prolifération et d'activité cellulaire, démontrant ainsi une stimulation de l'activité du follicule. D'autre part, l'hydrolysat selon l'exemple 1 diminue l'expression de p53 impliquée dans l'induction de la phase catagène.

### Exemple 4 : Mise en évidence de l'effet activateur de l'hydrolysat selon l'exemple 1 sur la matrice extracellulaire, sur les liaisons cellule-matrice et sur les liaisons cellule-cellule dans le follicule de cheveu

Le but de cette étude est de déterminer l'influence de l'hydrolysat selon l'exemple 1 sur l'expression des molécules de la matrice extracellulaire (fibronectine), des protéines de la lame basale (laminine-5) et des protéines impliquées dans les interactions cellule-cellule et cellule-matrice (intégrine bêta-1).

Protocole de culture : Des biopsies de peau humaine sont mises en culture dans les mêmes conditions que dans l'exemple 2, puis traitées pendant 48 heures par l'hydrolysat selon l'exemple 1 à 1 %. Des contrôles non traités sont également réalisés.

Protocole de l'immunomarquage de l'intégrine bêta 1 : A la fin de l'expérience, les biopsies sont mises en cassette et plongées dans un mélange de formol à 10 % pendant 2 heures dans un appareil automatisé (VIP). L'enrobage dans la paraffine est préparé par une série de bains d'alcool (à concentration et temps croissants), suivie de 2 bains de xylène et enfin d'un bain de paraffine. La durée totale de cette série d'opérations est d'une douzaine d'heures. Les biopsies incluses dans la paraffine sont ensuite coupées à 4 µm par un microtome et montées sur des lames. Les lames sont déparaffinées, réhydratées puis soumises à un immunomarquage par un anticorps monoclonal dirigé contre l'intégrine bêta 1 (Tebu, Santa Cruz), puis d'un anticorps secondaire adapté, couplé à un marqueur fluorescent. Les coupes de peau sont alors examinées au microscope à Epi-fluorescence (Nikon Eclipse E 80i microscope).

Protocole de l'immunomarquage de la laminine-5 (ou laminine 332 selon la nouvelle nomenclature) : A la fin de l'expérience, les biopsies sont incluses dans de la paraffine comme pour l'immunomarquage de l'intégrine bêta 1. Les lames sont ensuite déparaffinées, réhydratées puis soumises à une étape de démasquage préalable à l'immunomarquage par un anticorps monoclonal dirigé contre la laminine 332 (Chemicon). Un anticorps secondaire adapté, couplé à un marqueur fluorescent est ensuite mis en contact avec les coupes. Les coupes de peau sont enfin examinées au microscope à Epi-fluorescence (Nikon Eclipse E 80i microscope).

Protocole de l'immunomarquage de la fibronectine : A la fin de l'expérience les biopsies sont incluses dans de la résine OTC et congelées dans l'azote. Des coupes d'environ 6 µm sont ensuite réalisées au cryostat. Les coupes sont recueillies sur des lames d'observation poly-lysinées, puis les coupes sont fixées dans un bain d'acétone (conservé au préalable à - 20°C) pendant 10 minutes. L'immunomarquage est réalisé à l'aide d'un anticorps polyclonal de lapin spécifique (Sigma). Un anticorps secondaire adapté, couplé à un marqueur fluorescent est ensuite mis en contact avec les coupes. Les coupes de peau sont enfin examinées au microscope à Epi-fluorescence (Nikon Eclipse E 80i microscope).

Résultats : Sur les coupes de follicule de cheveu traitées par l'hydrolysat selon l'exemple 1, et dont la laminine -5 a été immunomarquée, on observe une fluorescence plus intense de la lame basale (zone de localisation de la laminine-5).

Sur les coupes de follicule de cheveu traitées par l'hydrolysat selon l'exemple 1, et dont la fibronectine a été immunomarquée, on observe une fluorescence plus intense de la de la matrice extracellulaire (zone de localisation de la fibronectine).

Sur les coupes de follicule de cheveu traitées par l'hydrolysat selon l'exemple 1, et dont l'intégrine bêta 1 a été immunomarquée, on observe une fluorescence plus intense du pourtour des cellules épithéliales de la gaine externe. A l'aide d'un logiciel de quantification de la fluorescence, on peut mesurer une augmentation de l'ordre de 244 % de l'intégrine bêta 1.

Conclusions : L'hydrolysat selon l'exemple 1 améliore la qualité de la matrice extracellulaire, l'adhésion cellule-cellule et l'adhésion cellule-matrice.

### Exemple 5 : Préparation de compositions

### 1 - Lait traitant antichute de cheveux :

Le produit est conçu pour être vaporisé sur le cuir chevelu et sur cheveux humides. Masser pour répartir uniformément le produit. Lutte contre la chute des cheveux tout en les rendant lisses et faciles à coiffer.

| **Formulation** | | **1** | **2** | |
|---|---|---|---|---|
| **Nom INCI** | **Nom commercial** | **% massique** | **% massique** | **Fournisseur** |
| Deionized Water | - | Q.S. | Q.S. | |
| Polyquaternium-11 | Gafquat® 755N | 1,25 | 2,00 | ISP |
| Propylene Glycol (and) Diazolidinyl Urea (and) Iodopropynyl Butylcarbamate/ | Liquid Germall® Plus | 0,50 | 0,50 | ISP |
| Sodium Polyacrylate (and) Hydrogenated Polydecene (and) Trideceth-6/ | RapiThix™ A-60 | 0,50 | 0,50 | ISP |
| | Hydrolysat selon l'exemple 1 | 0,1 | 0,5 | ISP |
| | Total | 100,00 | 100,00 | |

Mettre l'eau dans un récipient convenable et commencer l'agitation. Ajouter le Gafquat 755N et le Liquid Germall Plus et agiter jusqu'à obtenir un aspect uniforme. Ajouter le RapiThix A-60 et agiter jusqu'à obtenir un aspect uniforme (environ 15 minutes). Ajouter l'hydrolysat selon l'exemple 1 et agiter jusqu'à obtenir un aspect uniforme. Disposer le produit dans un vaporisateur non-aérosol équipé d'une pompe Calmar pump Mark VI WL31.

### 2 - Sérum pour la pousse des cheveux :

Appliquer le produit sur le cuir chevelu humide. Masser pour répartir uniformément le produit. Favorise la pousse ou repousse des cheveux tout en les rendant plus nerveux.

| **Formulation** | | **1** | **2** | |
|---|---|---|---|---|
| **Nom INCI** | **Nom commercial** | **% massique** | **% massique** | **Fournisseur** |
| Water | | Q.S. | Q.S. | |
| Hydroxyethylcellulose | Natrosol 250HHR | 0,35 | 0,50 | Hercules/Aqualon |
| Disodium EDTA | Dissolvine NA-2S | 0,05 | 0,05 | Akzo Nobel |
| VP/DMAPA Acrylates Copolymer | Styleze® CC-10 | 5,00 | 5,00 | ISP |
| Quaternium-26 | Ceraphyl® 65 | 1,00 | 1,00 | ISP |
| Panthenol | Ritapan DL | 0,15 | 0,15 | RITA |
| Propylene Glycol (and) Diazolidinyl Urea (and) Iodopropynyl Butylcarbamate | Liquid Germall® Plus | 0,50 | 0,50 | ISP |
| | Hydrolysat selon l'exemple 1 | 1,00 | 1,00 | ISP |
| Total | | 100,00 | 100,00 | |

Disperser le Natrosol 250HHR et le Disodium EDTA dans l'eau sous agitation. Chauffer à 50-60 °C, et agiter jusqu'à obtenir un aspect uniforme. Ajouter le Styleze® CC-10, et agiter jusqu'à obtenir un aspect uniforme. Laisser refroidir à température ambiante et ajouter les ingrédients dans l'ordre de la liste en agitant jusqu'à obtenir un aspect uniforme entre chacun d'eux.

### 3- Soin antiâge pour cheveu et cuir chevelu

Appliquer le produit sur le cuir chevelu humide. Masser pour répartir uniformément le produit. Lutte contre le vieillissement et la fragilisation des cheveux tout en les rendant lisses et faciles à coiffer.

| **Formulation** | | **1** | **2** | |
|---|---|---|---|---|
| **Nom INCI** | **Nom commercial** | **% massique** | **% massique** | **Fournisseur** |
| Phase A | | | | |
| Deionized Water | - | Q.S. | Q.S. | |
| Aminomethyl Propanol | AMP-95 | 0,05 | 0,05 | |
| Acrylic Acid/VP Crosspolymer | UltraThix™ P-100 | 0,85 | 0,85 | ISP |
| Phase B | | | | |
| Gycerol Dilaurate | Emulsynt™ GDL | 0,50 | 0,50 | ISP |
| Jojoba Seed Oil | - | 2,00 | 2,00 | Lipo |
| Cetearyl Alcohol | - | 2,00 | 2,00 | Rita |
| Phase C | | | | |
| Cyclopentasiloxane | SiTec™ CM040 | 0,50 | 1,00 | ISP |
| Phase D | | | | |
| VP/DMAPA Acrylates Copolymer | Styleze® CC-10 | 3,00 | 3,00 | ISP |
| Water | | 20,00 | 20,00 | |
| Aminomethyl Propanol | | 0,37 | 0,37 | |
| Phase E | | | | |
| Diazolidinyl Urea (and) Methylparaben (and) Propylene Glycol | Germaben® M | 0,75 | 0,75 | ISP |
| | Hydrolysat selon l'exemple 1 | 0,50 | 1,00 | ISP |
| Total | | 100,00 | 100,00 | |

Mettre l'eau et l'AMP-95 dans un récipient sous agitation. Ajouter l'UltraThix™ P-100 dans l'eau sous agitation vigoureuse et maintenir sous agitation pendant 30 minutes. Chauffer la phase A à 65 °C. Chauffer les ingrédients de la phase B à 65 °C puis les mélanger. Ajouter à la phase B et mélanger soigneusement. Refroidir à 35 °C. Ajouter la phase C au mélange principal et mélanger jusqu'à obtenir un aspect uniforme.

Indépendamment, mélanger les ingrédients de la phase D, jusqu'à obtenir un aspect uniforme. Ajouter le Germaben ®M (Phase E) et mélanger jusqu'à obtenir un aspect uniforme. Ajouter l'hydrolysat selon l'exemple 1 et agiter jusqu'à obtenir un aspect uniforme.

## Revendications

1. Utilisation cosmétique d'une composition comprenant au moins un hydrolysat peptidique de maïs (*Zea mays L.),* en tant qu'agent actif, pour stimuler et/ou restaurer la pousse du cheveu, ou prévenir la chute du cheveu.

2. Utilisation selon la revendication 1, **caractérisée en ce que** ledit hydrolysat peptidique comprend majoritairement des composés de nature peptidique ayant un poids moléculaire inférieur à 5 kDa.

3. Utilisation selon l'une des revendications précédentes, **caractérisée en ce que** ledit hydrolysat peptidique est solubilisé dans un ou plusieurs solvants physiologiquement acceptables, comme l'eau, le glycérol, l'éthanol, le propanediol, le butylène glycol, le dipropylène glycol, les diglycols éthoxylés ou propoxylés, les polyols cycliques ou tout mélange de ces solvants.

4. Utilisation selon l'une des revendications précédentes, **caractérisée en ce que** ledit hydrolysat peptidique contient entre 1 et 5 g/l, et préférentiellement entre 1,5 et 3,0 g/l de composés de nature peptidique.

5. Utilisation selon l'une des revendications précédentes, **caractérisée en ce que** ledit hydrolysat peptidique est utilisé en une quantité représentant de 0,001 % à 5 % du poids total de la composition, et préférentiellement en une quantité représentant de 0,01 % à 1 % du poids total de la composition.

6. Utilisation selon l'une des revendications précédentes, **caractérisée en ce que** la composition se présente sous une forme adaptée à l'application par voie topique comprenant un milieu physiologiquement acceptable.

7. Utilisation selon l'une des revendications précédentes, **caractérisée en ce que** la composition comprend en outre au moins un autre agent actif protecteur ou améliorant la pousse et/ou la santé des cheveux, destiné à favoriser et à compléter l'action dudit agent actif, choisi parmi des vitamines, des agents anti-radicalaires, anti-UV, des extrait peptidiques végétaux, le Minoxidil, des esters de l'acide nicotinique, des agents anti-inflammatoires, de l'acide rétinoïque ou ses dérivés, du rétinol, des inhibiteurs de la 5 alpha-réductase et des composés peptidiques issus de la synthèse chimique.

8. Utilisation selon la revendication précédente, **caractérisée en ce que** ledit autre agent actif est un extrait peptidique végétal.

9. Utilisation cosmétique dudit hydrolysat peptidique tel que défini dans l'une des revendications 1 à 4, en tant qu'agent actif stimulant le follicule de cheveu.

10. Utilisation selon la revendication 9, **caractérisée en ce que** l'agent actif augmente l'expression de la laminine-5, et/ou de l'intégrine bêta 1, et/ou de la fibronectine.

11. Utilisation selon la revendication 9, **caractérisée en ce que** l'agent actif augmente l'expression de la protéine p63, du marqueur Ki67 et la phosphorylation de l'histone 3, dans les cellules de la gaine épithéliale externe du follicule de cheveu.

12. Utilisation selon la revendication 9, **caractérisée en ce que** l'agent actif augmente l'élongation du cheveu.

13. Composition dermato-pharmaceutique comprenant un hydrolysat peptidique de maïs (*Zea mays L.)* en tant qu'agent actif capable de stimuler le follicule de cheveu pour son utilisation dans la stimulation et/ou la restauration de la pousse du cheveu ou dans la limitation de la chute du cheveu, lesquelles sont liées à un état pathologique.

14. Composition dermato-pharmaceutique pour son utilisation selon la revendication précédente, **caractérisée en ce que** les états pathologiques sont la pelade, les effets secondaires de traitements médicamenteux, certaines infections ou inflammations du cuir chevelu.

15. Composition dermato-pharmaceutique pour son utilisation selon la revendication 13, destinée au traitement pour lutter contre l'alopécie.

## Patentansprüche

1. Kosmetische Verwendung einer Zusammensetzung, umfassend mindestens ein Peptidhydrolysat von Mais (*Zea mays L.)* als aktive Substanz zur Stimulierung und/oder Wiederherstellung von Haarwuchs, oder zur Vorbeugung von Haarausfall.

2. Verwendung nach Anspruch 1, **dadurch gekennzeichnet, dass** das Peptidhydrolysat hauptsächlich Verbindungen peptidischer Art umfasst, aufweisend ein Molekulargewicht von weniger als 5 kDa.

3. Verwendung nach einem der vorhergehenden Ansprüche, **dadurch gekennzeichnet, dass** das Peptidhydrolysat in einem oder in mehreren physiologisch annehmbaren Lösemitteln solubilisiert ist, wie z. B. Wasser, Glycerol, Ethanol, Propandiol, Butylenglycol, Dipropylenglycol, ethoxylierten oder propoxylierten Diglycolen, cyclischen Polyolen oder jeder Mischung dieser Lösemittel.

4. Verwendung nach einem der vorhergehenden Ansprüche, **dadurch gekennzeichnet, dass** das Peptidhydrolysat zwischen 1 und 5 g/l und vorzugsweise zwischen 1,5 und 3,0 g/l Verbindungen peptidischer Art enthält.

5. Verwendung nach einem der vorhergehenden Ansprüche, **dadurch gekennzeichnet, dass** das Peptidhydrolysat in einer Menge verwendet wird, die 0,001 % bis 5 % des Gesamtgewichts der Zusammensetzung darstellt, und vorzugsweise in einer Menge, die 0,01 % bis 1 % des Gesamtgewichts der Zusammensetzung darstellt.

6. Verwendung nach einem der vorhergehenden Ansprüche, **dadurch gekennzeichnet, dass** die Zusammensetzung eine Form aufweist, die an die Verabreichung auf topischem Weg angepasst ist, umfassend ein physiologisch annehmbares Medium.

7. Verwendung nach einem der vorhergehenden Ansprüche, **dadurch gekennzeichnet, dass** die Zusammensetzung außerdem mindestens eine weitere aktive Substanz umfasst, die das Wachstum und/oder die Gesundheit der Haare schützt oder verbessert, die ausgelegt ist, um die Wirkung der aktiven Substanz zu fördern und zu vervollständigen, ausgewählt aus Vitaminen, Anti-radikalären- , Anti-UV-Substanzen, pflanzlichen Peptidextrakten, Minoxidil, Nikotinsäureestern, entzündungshemmenden Substanzen, Retinsäure oder ihren Derivaten, Retinol, Inhibitoren der 5-Alpha-Reduktase und Peptidverbindungen, die aus der chemischen Synthese stammen.

8. Verwendung nach dem vorhergehenden Anspruch, **dadurch gekennzeichnet, dass** die andere aktive Substanz ein pflanzlicher Peptidextrakt ist.

9. Kosmetische Verwendung des Peptidhydrolysats wie in einem der Ansprüche 1 bis 4 definiert, als aktive Substanz, die die Haarfollikel stimuliert.

10. Verwendung nach Anspruch 9, **dadurch gekennzeichnet, dass** die aktive Substanz die Expression von Laminin-5 und/oder von Integrin beta 1 und/oder von Fibronectin erhöht.

11. Verwendung nach Anspruch 9, **dadurch gekennzeichnet, dass** die aktive Substanz die Expression von Protein p63, des Markers Ki67 und der Phosphorylierung von Histon 3 in den Zellen der externen Epithelscheide des Haarfollikels erhöht.

12. Verwendung nach Anspruch 9, **dadurch gekennzeichnet, dass** die aktive Substanz die Verlängerung des Haars erhöht.

13. Dermopharmazeutische Zusammensetzung, umfassend ein Peptidhydrolysat von Mais (*Zea mays L*.) als aktive Substanz, das dazu in der Lage ist, das Haarfollikel zu stimulieren, für ihre Verwendung bei der Stimulierung und/oder Wiederherstellung des Haarwachstums oder bei der Begrenzung des Haarausfalls, die mit einem pathologischen Zustand verbunden sind.

14. Dermopharmazeutische Zusammensetzung für ihre Verwendung nach dem vorhergehenden Anspruch, **dadurch gekennzeichnet, dass** die pathologischen Zustände der kreisrunde Haarausfall, die Nebenwirkungen von Medikamentenbehandlungen, bestimmte Infektionen oder Entzündungen der Kopfhaut sind.

15. Dermopharmazeutische Zusammensetzung für ihre Verwendung nach Anspruch 13, ausgelegt zur Behandlung, um gegen die Alopezie zu kämpfen.

## Claims

1. Cosmetic use of a composition comprising at least one peptide hydrolysate of maize *(Zea mays L.),* as an active agent, in order to stimulate and/or restore hair growth or prevent hair loss.

2. Use according to claim 1, **characterised in that** said peptide hydrolysate comprises for the most part peptide compounds having a molecular weight of less than 5kDa.

3. Use according to one of the previous claims, **characterised in that** said peptide hydrolysate is solubilised in one or more physiologically acceptable solvents, such as water, glycerol, ethanol, propanediol, butylene glycol, dipropylene glycol, the ethoxylated or propoxylated diglycols, the cyclical polyols or any mixture of these solvents.

4. Use according to one of the previous claims, **characterised in that** said peptide hydrolysate contains between 1 and 5g/l, and preferably between 1.5 and 3.0g/l of peptide compounds.

5. Use according to one of the previous claims, **characterised in that** said peptide hydrolysate is used in a quantity representing from 0.001% to 5% of the total weight of the composition, and preferably in a quantity representing from 0.01% to 1% of the total weight of the composition.

6. Use according to one of the previous claims, **characterised in that** the composition is in a form suitable for topical use comprising a physiologically acceptable medium.

7. Use according to one of the previous claims, **characterised in that** the composition further comprises at least one other active agent that is protective or improves the growth and/or the health of hair, intended to promote and complement the action of said active agent, chosen from vitamins, anti-free-radical agents, anti-UV, plant peptide extracts, Minoxidil, esters of nicotinic acid, anti-inflammatory agents, retinoic acid or its derivatives, retinol, inhibitors of 5 alpha-reductase and peptide compounds made by chemical synthesis.

8. Use according to the previous claim, **characterised in that** said other active agent is a plant peptide extract.

9. Cosmetic use of said peptide hydrolysate as defined in one of claims 1 to 4, as an active agent stimulating hair follicles.

10. Use according to claim 9, **characterised in that** the active agent increases the expression of laminin-5, and/or of integrin beta 1, and/or of fibronectin.

11. Use according to claim 9, **characterised in that** the active agent increases the expression of the protein p63, of the marker Ki67 and the phosphorylation of histone 3, in the cells of the outer epithelial sheath of the hair follicle.

12. Use according to claim 9, **characterised in that** the active agent increases hair elongation.

13. Dermato-pharmaceutical composition comprising a peptide hydrolysate of maize *(Zea mays L.)* as an active agent capable of stimulating hair follicles for its use in the stimulation and/or the restoration of hair growth or in the limitation of hair loss, which are linked to a pathological state.

14. Dermato-pharmaceutical composition for its use according to the previous claim, **characterised in that** the pathological states are alopecia, the side effects of drug treatments, certain infections or inflammations of the scalp.

15. Dermato-pharmaceutical composition for its use according to claim 13, intended for the treatment for fighting alopecia.
